# EUROPEAN PATENT SPECIFICATION

(11) **EP 3 052 644 B1**
(45) Date of publication and mention of the grant of the patent: **10.07.2019**
(21) Application number: 14777631.4
(22) Date of filing: 01.10.2014
(51) Int. Cl.: C12Q 1/6806, C12Q 1/6844

(54) **DETECTION OF RARE MICROBIOLOGICAL NUCLEIC ACIDS**
DETEKTION VON SELTENEN MIKROBIOLOGISCHEN NUKLEINSÄUREN
DÉTECTION D'ACIDES NUCLÉIQUES MICROBIOLOGIQUES RARES

(30) Priority: 01.10.2013 EP 13306360
(43) Date of publication of application: 10.08.2016
(73) Proprietor: Texcell, 91000 Evry (FR)
(72) Inventor: DORANGE, Fabien, F-28630 Nogent Le Phaye (FR)
(74) Representative: Regimbeau
(86) International application number: PCT/EP2014/071025
(87) International publication number: WO 2015/049278

(56) References cited:
- CHARUL GIJAVANEKAR ET AL: "Rare target enrichment for ultrasensitive PCR detection using cot-rehybridization and duplex-specific nuclease", ANALYTICAL BIOCHEMISTRY, vol. 421, no. 1, 1 February 2012 (2012-02-01), pages 81-85, XP055092805, ISSN: 0003-2697, DOI: 10.1016/j.ab.2011.11.010
- J. CHEVAL ET AL: "Evaluation of High-Throughput Sequencing for Identifying Known and Unknown Viruses in Biological Samples", JOURNAL OF CLINICAL MICROBIOLOGY, vol. 49, no. 9, 29 June 2011 (2011-06-29), pages 3268-3275, XP055092780, ISSN: 0095-1137, DOI: 10.1128/JCM.00850-11 cited in the application
- BOGDANOVA EKATERINA A ET AL: "Normalization of full-length-enriched cDNA.", METHODS IN MOLECULAR BIOLOGY (CLIFTON, N.J.) 2011, vol. 729, 2011, pages 85-98, XP008166256, ISSN: 1940-6029
- EKATERINA A. BOGDANOVA ET AL: "Normalization of full-length enriched cDNA", MOLECULAR BIOSYSTEMS, vol. 4, no. 3, 1 January 2008 (2008-01-01) , page 205, XP055092920, ISSN: 1742-206X, DOI: 10.1039/b715110c
- YONG-LI XIAO ET AL: "High-throughput RNA sequencing of a formalin-fixed, paraffin-embedded autopsy lung tissue sample from the 1918 influenza pandemic", THE JOURNAL OF PATHOLOGY, vol. 229, no. 4, 15 February 2013 (2013-02-15), pages 535-545, XP055092926, ISSN: 0022-3417, DOI: 10.1002/path.4145

## Description

The present invention relates to a method as defined in the claims for the in vitro detection, in a biological sample, of at least one rare nucleic acid molecule from a micro-organism or a virus, wherein said method comprises a step of treatment of the nucleic acid molecules of said biological sample with a double-strand specific nuclease. The present invention also relates to a method as defined in the claims for the characterization of a biological product, and to a method for the evaluation of a manufacturing process for the preparation of a biological product, said methods comprising a step of treatment of the nucleic acid molecules with a double-strand specific nuclease.

The present invention also relates to the use as defined in the claims of a double-strand specific nuclease for the in vitro detection, in a biological sample, of a rare nucleic acid molecule of a micro-organism or a virus, for the characterization of a biological product and/or for the evaluation of a manufacturing process for the preparation of a biological product. The present invention also relates to a kit as defined in the claims for the detection of at least one rare nucleic acid from a micro-organism or a virus.

Molecular methods became essential tools in diagnostic laboratories with the use of real time PCR, or qPCR, as an amplification and detection method. As a powerful tool for diagnostic, questions arise if molecular biology methods should replace traditional methods for routine diagnostic such as cell culture for virology and culture media for bacteria. In the field of virology, qPCR have been used for the detection and analyses of viruses that cannot growth in cell culture such as, but not limited to, HCV, HBV and B19. This method has improved sensitivity and rapidity of the detection. Although the design of the qPCR is driven by the amplification of highly conserved regions, there are some limitations of this technology to detect all genomes in a genus or new mutated genome. For example, a set of 9 qPCR is necessary for the detection of Human Immunodeficiency virus type 1 (HIV-1) strains (Gardner *et al.,* 2003). Moreover, the detection of different amplified sequences in a multiplex qPCR is limited to 4 or 5 targets. Thus, when an exhaustive control is needed, a high quantity of sample is needed to perform all qPCR assays.

High Throughput Sequencing (HTS), also named Next-Generation Sequencing (NGS), has been widely used for whole genome sequencing and in metagenomic studies. In the microbiology field, it has been applied for the identification of genome variability within the host or a population, but the main application of this method has been its successful use for the detection and discovery of new pathogens. The strength and the main advantage of NGS is its capacity to detect the full spectrum of micro-organisms (Barzon *et al.,* 2011) without an "a priori" testing. Other advantage is the direct identification of the pathogen if the detected sequences are present in an available database such as Genbank.

Other sequencing based methods were successfully applied for the detection of new pathogens, including subtractive methods, sequencing of nuclease resistant protected fragments, but these methods are laborious and not sensitive enough for high throughput analysis.

New methods such as microarray methods and electrospray Ionization Mass spectroscopy (PCR/ESI-MS), relying on base composition signatures obtained from PCR amplification of broadly conserved regions of viral genomes, were developed in order to achieve a wider viral detection. Although these methods are designed for high broad detection and the discovery of new viruses in a viral family, they are not anticipated to allow the detection of whole pathogens due to their sequence specificity.

In NGS assay, sensitivity is driven by the quantity of the nucleic acid molecules. As NGS sequences all nucleic acid molecules in a sample, increasing the sensitivity relies on decreasing the level of non-interesting sequences which, in the case of a microbiological diagnostic, is nucleic acid molecules from the host. To detect the lowest level of contaminant in a biological sample, the greatest possible number of copies of a sequence, or "reads", need to be generated. However, in some cases, said greatest number may not be sufficient to detect a contaminant.

Recent advances were made in order to improve the sensitivity of the NGS assay and to overcome the high level of predominant nucleic acids. A number of documents describe the treatment of samples with nucleases hydrolysing free nucleic acid present in a sample, while the viral nucleic acid is protected. However the non-accessibility of nucleases to nucleic acid molecules tightly linked to proteins makes this hydrolysis step not sufficient.

A modified VIDISCA method (Virus discovery based on cDNA-AFLP (amplified fragment length polymorphism)) was used in order to decrease the amount of rRNA in a sample (Vries *et al.,* 2010). Also, Cheval *et al.* (2011) describe the use of rolling circle amplification (RCA) using Phi 29 in a MRC5 supernatant spiked with different viruses at a concentration near the LOD (Limit of Detection). As disclosed in Cheval *et al.,* the Phi29 amplification increases the sensitivity of HTS samples and detects 7 out of 9 viruses with a spike at 3 x LOD and 4 out of 9 viruses with a spike at 0.8 x LOD. However comparative assays demonstrate the highest sensitivity of qPCR.

Cheval et al. (Journal of clinical microbiology, vol.49, n°9, 29 June 2011) discloses the use of HTS technology to detect different virus simultaneously in a given biological sample, but this document does not disclose DSN treatment.

The use of DSN treatment is known from Charul Gijavanekar et al. (Analytical biochemistry, vol.421, n°1, 1 February 2012) in a method for detecting rare target nucleic acids with a sensitive PCR, but this document uses cDNA fragments from plasmids PUC and not a biological sample as claimed in the present invention.

Therefore, there is still a need to improve the sensitivity of rare nucleic acids detection methods.

This is the object of the present invention.

The present invention relates to a method based on the decrease of predominant nucleic acid molecules in a sample comprising nucleic acids, due to the use of a duplex specific nuclease (DSN) at the pre-amplification level, which can act as a normalized method to enhance rare nucleic acids for the in vitro preparation of a sample comprising nucleic acid molecules, wherein said sample is treated with a double-strand specific nuclease before any amplification step.

A recent publication describes a DSN treatment at the post amplification level only, in order to normalize the population of nucleic acid molecules during the library preparation and has been shown to decrease the variability in genome coverage (Rodrigue *et al.,* 2009). It is to be noted that, conversely to the present invention, the goal of a method according to Rodrigue et al., and the problem solved by said method, is to avoid as much as possible the sequencing of rare contaminating nucleic acids. Ekaterina Bogdanova et al. (Molecular BioSystems, vol.4, n°3, 1 January 2008; and Methods in molecular biology, vol.729, 2011) also disclose the use of DSN in a method for the generation of normalized cDNA libraries, but DSN is used in this document after the preparation of DNA library, which means after the step of DNA amplification, which is the reverse in the present invention.

In a method according to the invention, a treatment with a double-strand specific nuclease relatively decreases the amount of predominant double stranded DNA molecules and increases the proportion of rare nucleic acid molecules in the sample.

A method according to the invention allows to lower the bias in the generation of DNA libraries and to reach sensitivity similar or superior to that of qPCR, which is the most sensitive assay to date. A method according to the present invention presents the following advantages:
- Non "a priori" testing, no need of a sequence-specific primer for the detection
- Allows the identification of the pathogen
- As sensitive as qPCR

The present invention is directed to a method as defined in the claims for the detection with high sensitivity of a micro-organism or a viral agent in a biological sample, such as a cell supernatant, said method comprising a step of treatment of the nucleic acid molecules with a double-strand specific nuclease. This protocol is designed for the detection of any micro-organism or virus, enveloped or naked, single or double stranded DNA or RNA. A method of the present invention provides a general scheme that can be applied to the detection with a high sensitivity of rare nucleic acid molecule from any microbiological or viral agent.

The present invention will become more fully understood from the detailed description and disclosure given herein and from the accompanying drawings, which are given by way of illustration only and do not limit the intended scope of the invention.

The present invention relates to the subject of the claims.

The present disclosure further relates to a method for the in vitro detection in a biological sample of the presence of at least one rare nucleic acid from a micro-organism or a virus, said method comprising the steps of:
a) extracting the nucleic molecules from a biological sample, wherein said nucleic acid molecules include double strand nucleic acid molecules, single strand nucleic acid molecules or a combination thereof,
b) inducing the denaturation and the renaturation of the double strand nucleic acid molecules of step a),
c) contacting the product of step b) with a duplex specific nuclease (DSN),
d) inactivating and/or extracting said DSN from the product of step c) and concentrating of the nucleic acid molecules,
e) amplifying the nucleic acid molecules of the product of step d),
f) preparing a DNA library from the product of step e),
g) determining the nucleic acid sequence of molecules of the DNA library of step f) and detecting, from said nucleic acid sequence, the presence of at least one rare nucleic acid from a micro-organism or a virus.

The term "biological sample" designates in the present disclosure any sample or extract susceptible to comprise, or comprising, any biological material. More preferably, a biological sample in a method disclosed herein comprises nucleic acid molecules and even more preferably, a biological sample in a method disclosed herein comprises nucleic acid molecules and cells.

In particular embodiment, the present invention relates to a method for the in vitro detection in a biological sample of at least one rare nucleic acid from a micro-organism or a virus, wherein said biological sample is prepared from a material chosen in the group consisting of: a human biological fluid or tissue, an animal biological fluid or tissue, a culture of a human or an animal biological fluid or tissue, a culture of eukaryotic cells, a culture of prokaryotic cells, a culture of both eukaryotic and prokaryotic cells. A biological sample is prepared from a biological material by the use of methods such as the extraction or the purification of the biological sample to be adapted for performing a method according to the invention.

By "biological fluid", it is intended a fluid is defined as a liquid originating from the body and possibly excreted or secreted. In a method according to the present invention, said body fluid or tissue is chosen in the group consisting of: plasma, serum, whole blood, whole blood extract, urine, feces, sweat, lymph, amniotic fluid, bile, breast milk, saliva, sputum, tears, semen, vaginal secretion, aqueous humour, vitreous humour and cerebrospinal fluid.

In a particular embodiment, the present invention relates to a method for the in vitro detection in a biological sample of at least one rare nucleic acid from a micro-organism or a virus, wherein said biological sample is a bio-therapeutic product, preferably chosen in the group consisting of: recombinant protein, monoclonal antibody, vaccine, cell therapy product, gene therapy product, products of human or animal origin such as blood-derived products, heparin, hyaluronic acid and collagen.

In another particular embodiment, the invention relates to a method for the in vitro detection in a biological sample of at least one rare nucleic acid from a micro-organism or a virus, wherein the culture of a human or an animal biological fluid or tissue comprises cells from a cell culture, wherein said cell culture comprises eukaryotic cells, prokaryotic cells or a combination thereof. Eukaryotic cells include human, animal, plants, fungi and unicellular eukaryotes such as yeasts. Prokaryotic cells include bacteria as well as archea. In a more particular embodiment, in a method for the in vitro detection in a biological sample of at least one rare nucleic acid from a micro-organism or a virus, according to the invention, a biological sample is chosen in the group consisting of: cells from a cell culture infected by a virus, cells from a hybridoma production such as mouse/human hybridomas used for the production of monoclonal antibodies, and cells susceptible to be used for the production of a recombinant protein. In another particular embodiment, the invention relates to a method for the in vitro detection in a biological sample of at least one rare nucleic acid from a micro-organism or a virus, wherein said biological sample is a sample from a cell bank, preferably chosen in the group consisting of: a master cell bank, a working cell bank and a production cell bank.

By "nucleic acid", it is intended to designate polymer molecules comprising natural or artificial nucleotides, and preferably deoxyribonucleic acid (DNA) and/or ribonucleic acid (RNA). According to a method of the invention, a "rare nucleic acid molecule" is a molecule which is present in a sample in a limited proportion or frequency, in concentration of bases of this nucleic acid molecule, comparatively to the total amount of nucleic acid molecules comprised in said biological sample. In a particular embodiment, the present invention relates to a method for the detection in a biological sample of at least one rare nucleic acid molecule, wherein, when compared to the total amount of nucleic acid molecules, said at least one rare nucleic acid molecule is present at a proportion comprised between about 1/10⁴ and about 1/10⁹, preferably between about 1/10⁵ and about 1/10⁸, more preferably between about 1/10⁶ and about 1/10⁷, wherein "about" is intended to mean +/- 10%. In a particular embodiment of a method according to the invention, a "rare nucleic acid molecule" is a nucleic acid molecule of a micro-organism or of a virus defined as a function of the limit of detection (LOD) of said micro-organism or a virus.

By "micro-organism", it is intended a microbial agent or a microscopic organism chosen among eukaryotes and prokaryotes.

In a method for the in vitro detection in a biological sample of the presence of at least one rare nucleic acid from a micro-organism or a virus, according to the invention, the extraction of nucleic acid molecules from a biological sample can be performed by any of the methods described for isolating nucleic acid molecules, such as methods described in Maniatis T. *et al.* (Edition 1999), well known by a person skilled in the art of manipulating nucleic acid molecules and biological products comprising said nucleic acid molecules.

Preferably, the extraction of the nucleic acid is performed by selecting an extraction procedure that is highly efficient even in the absence of carrier molecule such as RNA carrier. As non-limiting examples of kits leading to an adapted recovery, Silica-adembeads (Ademtech) and NucleoSpin RNA virus (Macherey Nagel) may be used. Preferably, extracting the nucleic acid molecules is performed in the absence of any molecule defined as a RNA carrier, which are used in some protocols for purifying and concentrating nucleic acids. Indeed, the RNA carrier can act as a matrix in further steps and be a main contaminant sequence in further HTS.

By "nucleic acid denaturation" is intended the step wherein nucleic acid complementary strands, or nucleic acid complementary structures in a single nucleic acid strand, are dissociated. Double strand nucleic acid molecules and double strand nucleic acid structures are formed by non-covalent association through hydrogen-bonds between complementary nucleotides, nucleic acid denaturation occurs by the breakage of said hydrogen-bonds. The nucleotide composition of a strand may influence the conditions for denaturation-renaturation, as G and C bases may be linked by 3 hydrogen bonds and A and T bases may be linked by 2 hydrogen bonds. Strands comprising a high proportion of G and C bases will be less easily submitted to denaturation than a strand comprising a lower proportion of G and C bases. Nucleic acids denaturation is reversible. A person skilled in the art will easily determine the conditions for inducing denaturation and renaturation. In a method according to the invention, inducing the denaturation of double strand nucleic acid molecules can be achieved by any method submitting nucleic acids to any physical or chemical agent able to destabilize hydrogen bonds, such as a raised temperature, pH, solvents, high ionic concentration and alcalinic agents. An enzymatic agent may also be able to induce said hydrogen-bonds breakage and denaturation. An example of a usable agent is a chaotropic element.

Nucleic acid denaturation is reversible, when denaturation has been induced by raising the temperature, a step of temperature cooling allows renaturation, wherein complementary strands re-assemble with formation of hydrogen-bonds. When denaturation has been induced by applying a particular condition or adding a particular agent, any method leading to the extraction of the nucleic acid from said agent may allow the renaturation of said nucleic acid.

In a particular embodiment, a method according to the invention for the in vitro detection of a rare nucleic acid from a micro-organism or a virus comprises a step of inducing the denaturation of nucleic acids molecules wherein nucleic acid samples are placed at a temperature comprised between about 85 °C and about 105 °C, preferably between about 90 °C and about 100 °C, and preferably about 95 °C. The term "about" designates a possible variation of more or less 10%. After double strand denaturation induced by heating the sample, the renaturation step comprises the progressive return back to the initial temperature, to allow the re-association of complementary double strand nucleic acids, during an annealing step. These methods are well known by a person skilled in the art. In a particular embodiment, a method according to the invention for the in vitro detection of a rare nucleic acid from a micro-organism or a virus, comprises a step of inducing the renaturation of nucleic acid molecules wherein nucleic acid are placed at a temperature of about 70°C for the time necessary for renaturation, preferably for at least 4 hours. Nucleic acids denaturation and renaturation may be performed in a thermal cycler and may be easily monitored by UV spectroscopy at 260 nm.

In a particular embodiment of a method for the detection of at least one rare nucleic acid from a micro-organism or a virus, inducing the denaturation of the double strand nucleic acid molecules is achieved by any method known by a person skilled in the art to induce said denaturation, preferably by submitting said nucleic acid sample to an elevated temperature.

In a more particular embodiment, a method according to the invention for the in vitro detection of a rare nucleic acid from a micro-organism or a virus, comprises a step of inducing the denaturation of the double-strand nucleic acid molecules wherein nucleic acid are placed at a temperature of about 95 °C, followed by a step of inducing the renaturation of the double-strand nucleic acid molecules wherein nucleic acid are placed at a temperature of about 70 °C for at least 4 hours.

For nucleic acid molecules placed for 4 hours at about 70°C, the temperature of about 70°C ensures that renaturation occurs in a very specific manner and limits wrong pairing.

A "duplex specific nuclease" or "double strand specific nuclease" is an enzyme that specifically cleaves double strand or paired DNA molecules, or double strand or paired DNA structures in a DNA molecule, and that cleaves paired nucleic acid molecules consisting of a paired DNA and RNA molecules.

The treatment with a duplex specific nuclease leads to the specific cleavage of DNA molecules in double stranded DNA and in DNA-RNA hybrid duplexes, and does not noticeably cleave single strand DNA. As a particular example, DSN purified from hepatopancreas of Red King (Kamchatka) crab (Shagin *et al.,* 2002) can be cited. DSN also discriminates between perfectly and nonperfectly matched short DNA duplexes. DSN is widely used for full-length enriched cDNA normalization (Zhulidov *et al.,* 2004; Bogdanova *et al.,* 2011a) and for construction of normalized RNA-seq libraries for next generation sequencing (Christodoulou *et al.,* 2011).

The concentration of the nucleic acid molecules corresponds to concentrating the nucleic acid molecules until their concentration is of at least 2 times the initial concentration. This concentration is adapted to the performance of the subsequent enzymatic reactions.

As a non-limiting example, the concentration of the nucleic acid molecules may be performed via the use of an extraction kit, such as smart D-N Ademkit. This procedure efficiently removes the DSN enzyme and concentrates the nucleic acids. The DSN is inactivated or eliminated by any method known to inactivate or eliminate an enzyme, such as heating or physically extracting the enzyme. As an example, DSN may be extracted by using the extraction procedure smart D-N Ademkit.

The nucleic acid molecules are amplified by using any method known by a person skilled in the art. Preferably, the amplification of the nucleic acids is performed by the rolling circle amplification (RCA). DNA fragments are firstly ligated and then amplified by the Phi29 enzyme. The Phi 29 enzyme is not efficient for processing small genomes if the number of genome copies is close to or inferior to 1000 genomes. The ligation step is performed to ligate these small fragments so that they can be amplified in the RCA amplification. Any commercial kit for RCA may be used for this step. Preferably, the RCA procedure is performed according to the qiagen® WTA kit.

A DNA library is a collection of DNA or cDNA that represents the known DNA or cDNA sequences from a particular organism or sample. In a method for the in vitro preparation of a DNA library according to the invention, a DNA library is preferably a cDNA library, comprising cDNA fragments obtained from reverse transcription of RNA molecules. In NGS, DNA or cDNA fragments can directly be used as the library for clonal amplification if necessary.

Among numerous methods for determining the sequence of a nucleic acid molecule, including chain termination (Sanger sequencing), the following next-generation sequencing (NGS) methods may be cited: Single-molecule real-time sequencing (Pacific Bio®), Ion semiconductor (Ion Torrent sequencing) Pyrosequencing (454) Sequencing by synthesis (Illumina ®) and sequencing by ligation (SOLiD sequencing).

In a particular embodiment, the present invention relates to a method for the in vitro detection of the presence of at least one rare nucleic acid from a micro-organism or a virus in a sample wherein, in step a), nucleic acid molecules comprise DNA molecules, RNA molecules or a combination thereof, and wherein the extraction of the nucleic acid molecules is followed by the reverse transcription of the RNA molecules of step a).

The expression "reverse transcription of the RNA" defines the method wherein RNA molecules of the sample are contacted with a reverse transcriptase enzyme in conditions adapted for the generation of a cDNA molecule from the RNA molecules. Any reverse transcriptase may be used in a method according to the invention, however transcriptase RNase H minus or transcriptase with reduced RNase H activity are preferred. As a non-limiting example, reverse transcription of the RNA molecules is performed using engineering or non-modified reverse transcriptase (RT), more preferably superscript III RT (Life technologies).

In a particular embodiment, the present invention relates to a method for the in vitro detection of at least one rare nucleic acid from a micro-organism or a virus in a sample wherein the DNA library of step f) is treated with a DSN, before performing step g).

In a particular embodiment, the present invention relates to a method for the detection of at least one rare nucleic acid molecule in a sample wherein said at least one rare nucleic acid molecule is a nucleic acid molecule of a micro-organism.

In a method according to the invention, a micro-organism, or a microbial agent, is a microscopic organism chosen among eukaryotes, such as yeasts, fungi, algae, protozoa, and prokaryotes, such as bacteria and archea. Bacteria include Gram positive and Gram negative bacteria. In a more particular embodiment, the present invention relates to a method for the detection of at least one microbial agent, wherein said microbial agent is chosen in the group consisting of: Gram positive bacteria, gram negative bacteria, mycobacteria, mycoplasmas and fungi. In a particular embodiment of a method for the detection of a rare nucleic acid molecule, the nature of the genome of said micro-organism is chosen in the group consisting of: double-strand DNA, single-strand DNA, double-strand RNA and single-strand RNA.

In a particular embodiment, the present invention relates to a method for the detection of the presence of at least one rare nucleic acid from a mycoplasma. In a more particular embodiment, the present invention relates to a method for the detection of the presence of at least one rare nucleic acid from a mycoplasma chosen in the group consisting of: *Mycoplasma hyorhinis*, *Mycoplasma pneumoniae*, *Acheloplasma laidlawii*, *Mycoplasma fermentans*, *Mycoplasma orale*, *Spiroplasma citri*, *Mycoplasma synoviae*, *Mycoplasma gallisepticum* and *Mycoplasma arginini.*

In a more particular embodiment, the present invention relates to a method for the detection of the presence of at least one rare nucleic acid from *Mycoplasma orale.*

In another particular embodiment, the present invention relates to a method for the detection of at least one rare nucleic acid molecule in a sample wherein said at least one rare nucleic acid molecule is a nucleic acid molecule of a virus.

In a particular embodiment, the present invention relates to a method for the detection of at least one rare nucleic acid molecule from a virus in a sample wherein the genome of said virus is encapsulated. In another particular embodiment, the present invention relates to a method for the detection of at least one rare nucleic acid molecule from a virus in a sample wherein the genome of said virus is free.

In a more particular embodiment, the present invention relates to a method for the detection of at least one rare nucleic acid molecule from a virus in a sample wherein said virus is chosen in the group consisting of: double-strand DNA virus, single-strand DNA virus, double-strand RNA virus and single-strand RNA virus. In an even more particular embodiment, the present invention relates to a method for the detection of at least one rare nucleic acid molecule from a virus in a sample, wherein said virus is a "segmented virus", whose genome is divided in separate parts. In another more particular embodiment, the present invention relates to a method for the detection of at least one rare nucleic acid molecule from a virus in a sample, wherein said virus is a "non-segmented virus", whose genome is not divided in separate parts.

In a more particular embodiment, the present invention relates to a method for the detection of at least one rare nucleic acid molecule from a virus in a sample wherein said virus is chosen in the group consisting of: human virus, simian virus, rodent virus, equine virus, bovine virus, porcine virus, insect virus and bacteriophages.

In a particular embodiment, the present invention relates to a method for the in vitro detection in a biological sample of at least one rare nucleic acid from a virus, wherein said virus is chosen in the group consisting of:
- Adenoviridae (including adenovirus 5)
- Anelloviridae
- Bornaviridae
- Bunyaviridae
- Caliciviridae (including vesivirus 2117)
- Circoviridae (including Porcine circovirus type 1 and 2)
- Coronaviridae
- Filoviridae
- Flaviviridae (including BVDV, Hepatitis C virus, West Nile virus)
- Hepadnaviridae (including Hepatitis B virus)
- Hepeviridae (including Hepatitis E virus)
- Herpesviridae (including HSV, HCMV, EBV, HHV-6, HHV-7, HHV-8)
- Orthomyxoviridae
- Papillomaviridae
- Paramyxoviridae (including RSV-A, RSV-B)
- Parvoviridae (including B19, MVM, PPV)
- Picornaviridae (including Hepatitis A virus)
- Polyomaviridae (including human polyomavirus BK and JC, MyCV)
- Poxviridae
- Reoviridae
- Retroviridae (including Human Immunodeficiency virus, Murine leukemia virus)
- Rhabdoviridae, and
- Togaviridae

In a more particular embodiment, the present invention relates to a method for the in vitro detection in a biological sample of at least one rare nucleic acid from a virus, wherein said virus is chosen in the group consisting of: bovine viral diarrhoea virus (BVDV); bovine adenovirus type 3 (BAV3); bovine parvovirus (BPV); bluetongue virus (BTV), BT-2 strain; bovine syncytial virus (BRSV), TN strain; reovirus type 3 (Reo3), Abney strain MLV PPV, influenza B, FluA H3N8 and rabies virus.

In a more particular embodiment, the present invention relates to a method for the in vitro detection in a biological sample of at least one rare nucleic acid from a virus, wherein the specific detection of said virus is performed by using at least one, preferably two, specific primers, wherein said primers are chosen in the group consisting of:
a) For detecting a nucleic acid from Ad5: a primer comprising a nucleotide sequence identical or having at least 90% identity with a sequence chosen among SEQ ID N°1, SEQ ID N°2, and SEQ ID N°3.
b) For detecting a nucleic acid from BK: a primer comprising a nucleotide sequence identical or having at least 90% identity with a sequence chosen among SEQ ID N°4, SEQ ID N°5, and SEQ ID N°6.
c) For detecting a nucleic acid from BVDV: a primer comprising a nucleotide sequence identical or having at least 90% identity with a sequence chosen among SEQ ID N°7, SEQ ID N°8, and SEQ ID N°9.
d) For detecting a nucleic acid from HBV: a primer comprising a nucleotide sequence identical or having at least 90% identity with a sequence chosen among SEQ ID N°10, SEQ ID N°11, and SEQ ID N°12.
e) For detecting a nucleic acid from FluA: a primer comprising a nucleotide sequence identical or having at least 90% identity with a sequence chosen among SEQ ID N°13, SEQ ID N°14, and SEQ ID N°15.
f) For detecting a nucleic acid from MLV: a primer comprising a nucleotide sequence identical or having at least 90% identity with a sequence chosen among SEQ ID N°16, SEQ ID N°17, and SEQ ID N°18.
g) For detecting a nucleic acid from PPV: a primer comprising a nucleotide sequence identical or having at least 90% identity with a sequence chosen among SEQ ID N°19, SEQ ID N°20, and SEQ ID N°21.
h) For detecting a nucleic acid from Reo3: a primer comprising a nucleotide sequence identical or having at least 90% identity with a sequence chosen among SEQ ID N°22, SEQ ID N°23, and SEQ ID N°24.

The present invention also relates to a method for the characterization for the presence and/or the level of at least one micro-organism or virus of a biological sample, said method comprising a method according to the invention for the in vitro detection in said biological sample of at least one rare nucleic acid from a micro-organism or a virus, and the characterization of said biological sample for the presence and/or the level of said at least one micro-organism or virus.

By "characterization for the presence and/or the level of at least one micro-organism or virus" is intended the determination of the presence or absence of at least one micro-organism or virus and/or, if present, the quantity of said least one micro-organism or virus. In a particular embodiment, a method of the present invention for the characterization for the presence and/or the level of at least one micro-organism or virus of a biological product, is a method for the determination of the presence and/or level of a contaminating micro-organism or virus in a biological product.

In a particular embodiment, the present invention relates to a method for the characterization for the presence and/or the level of at least one micro-organism of a biological product, said method comprising a method according to the invention for the in vitro detection in a biological sample of at least one rare nucleic acid from said micro-organism, and the characterization of said biological product for the presence and/or the level of said at least one micro-organism, wherein said micro-organism is chosen in the group of: Gram positive bacteria, Gram negative bacteria, mycoplasma, yeast and fungi.

In a more particular embodiment, the present invention relates to a method for the characterization for the presence and/or the level in a biological product of at least one micro-organism, said method comprising a method according to the invention for the in vitro detection in a said biological product of at least one rare nucleic acid from said micro-organism, and the characterization of said biological product for the presence and/or the level of said at least one micro-organism, wherein said micro-organism is a mycoplasma chosen in the group consisting of: *Mycoplasma hyorhinis*, *Mycoplasma pneumoniae*, *Acheloplasma laidlawii*, *Mycoplasma fermentans*, *Mycoplasma orale*, *Spiroplasma citri*, *Mycoplasma synoviae*, *Mycoplasma gallisepticum* and *Mycoplasma arginini.*

In another particular embodiment, the present invention relates to a method for the characterization for the presence and/or the level in a biological product of at least one virus, said method comprising a method according to the invention for the in vitro detection in said biological product of said at least one rare nucleic acid from said virus, and the characterization of said biological product for the presence and/or the level of said at least one virus, wherein said virus is chosen in the group of: Adenoviridae, Anelloviridae, Bornaviridae, Bunyaviridae, Caliciviridae, Circoviridae, Coronaviridae , Filoviridae, Flaviviridae, Hepadnaviridae, Hepeviridae, Herpesviridae, Orthomyxoviridae, Papillomaviridae, Paramyxoviridae, Parvoviridae, Picornaviridae, Polyomaviridae, Poxviridae, Reoviridae, Retroviridae, Rhabdoviridae, and Togaviridae.

In another more particular embodiment, the present invention relates to a method for the characterization for the presence and/or the level in a biological product of at least one virus, said method comprising a method according to the invention for the in vitro detection in said biological product of said at least one rare nucleic acid from said virus, and the characterization of said biological product for the presence and/or the level of said at least one virus, wherein said virus is chosen in the group consisting of: adenovirus 5, vesivirus 2117, Porcine circovirus type 1 and 2, Hepatitis C virus, West Nile virus, Hepatitis B virus, Hepatitis E virus, HSV, HCMV, EBV, HHV-6, HHV-7, HHV-8, RSV-A, RSV-B, B19, MVM, PPV, Hepatitis A virus, human polyomavirus BK and JC, MyCV, Human Immunodeficiency virus, Murine leukemia virus, bovine viral diarrhoea virus (BVDV); bovine adenovirus type 3 (BAV3); bovine parvovirus (BPV); bluetongue virus (BTV), BT-2 strain; bovine syncytial virus (BRSV), TN strain; reovirus type 3 (Reo3), Abney strain; MLV; PPV; influenza B;, FluA H3N8; and rabies virus.

In another particular embodiment, a method of the present invention for the characterization for the presence and/or the level of at least one micro-organism or virus of a biological product, is a method for the evaluation of the safety of a biological product, wherein the determination of the presence and/or the level of said at least one micro-organism or virus is compared to the known acceptable presence and/or level of a given micro-organism or virus in a particular biological product.

In another particular embodiment, the present invention relates to a method for the evaluation of a manufacturing process for the preparation of a biological product, said method comprising a method for the in vitro detection of at least one rare nucleic acid from a micro-organism or a virus in a biological sample according to the invention, wherein said in vitro detection in said biological product of at least one rare nucleic acid from a micro-organism or a virus is performed at least two different stages of said manufacturing process.

In a more particular embodiment, the present invention relates to a method for the evaluation of a manufacturing process for the preparation of a biological product, said method comprising a method according to the invention for the in vitro detection in said biological product of at least one rare nucleic acid from a micro-organism, wherein said micro-organism is chosen in the group consisting of: Gram positive bacteria, Gram negative bacteria, mycoplasma, yeast and fungi.

In an even more particular embodiment, the present invention relates to a method for the evaluation of a manufacturing process for the preparation of a biological product, said method comprising a method according to the invention for the in vitro detection in said biological product of at least one rare nucleic acid from a micro-organism, wherein said micro-organism is a mycoplasma chosen in the group consisting of: *Mycoplasma hyorhinis*, *Mycoplasma pneumoniae*, *Acheloplasma laidlawii*, *Mycoplasma fermentans*, *Mycoplasma orale*, *Spiroplasma citri*, *Mycoplasma synoviae*, *Mycoplasma gallisepticum* and *Mycoplasma arginini.*

In another more particular embodiment, the present invention relates to a method for the evaluation of a manufacturing process for the preparation of a biological product, said method comprising a method according to the invention for the in vitro detection in said biological product of at least one rare nucleic acid from a micro-organism, wherein said micro-organism is a virus chosen in the group consisting of: Adenoviridae, Anelloviridae, Bornaviridae, Bunyaviridae, Caliciviridae, Circoviridae, Coronaviridae , Filoviridae, Flaviviridae, Hepadnaviridae, Hepeviridae, Herpesviridae, Orthomyxoviridae, Papillomaviridae, Paramyxoviridae, Parvoviridae, Picornaviridae, Polyomaviridae, Poxviridae, Reoviridae, Retroviridae, Rhabdoviridae, and Togaviridae.

In another more particular embodiment, the present invention relates to a method for the evaluation of a manufacturing process for the preparation of a biological product, said method comprising a method according to the invention for the in vitro detection in said biological product of at least one rare nucleic acid from a micro-organism, wherein said micro-organism is a virus chosen in the group consisting of: adenovirus 5, vesivirus 2117, Porcine circovirus type 1 and 2, Hepatitis C virus, West Nile virus, Hepatitis B virus, Hepatitis E virus, HSV, HCMV, EBV, HHV-6, HHV-7, HHV-8, RSV-A, RSV-B, B19, MVM, PPV, Hepatitis A virus, human polyomavirus BK and JC, MyCV, Human Immunodeficiency virus, Murine leukemia virus, bovine viral diarrhoea virus (BVDV); bovine adenovirus type 3 (BAV3); bovine parvovirus (BPV); bluetongue virus (BTV), BT-2 strain; bovine syncytial virus (BRSV), TN strain; reovirus type 3 (Reo3), Abney strain MLV PPV, influenza B, FluA H3N8 and rabies virus.

In another particular embodiment, the present invention relates to a method for the characterization for the presence and/or the level in a biological product of at least one virus, said method comprising a method according to the invention for the in vitro detection in said biological product of said at least one rare nucleic acid from said virus, and the characterization of said biological product for the presence and/or the level of said at least one virus, wherein said virus is chosen in the group of: Adenoviridae, Anelloviridae, Bornaviridae, Bunyaviridae, Caliciviridae, Circoviridae, Coronaviridae , Filoviridae, Flaviviridae, Hepadnaviridae, Hepeviridae, Herpesviridae, Orthomyxoviridae, Papillomaviridae, Paramyxoviridae, Parvoviridae, Picornaviridae, Polyomaviridae, Poxviridae, Reoviridae, Retroviridae, Rhabdoviridae, and Togaviridae.

In another particular embodiment, the present invention relates to a method for the characterization for the presence and/or the level in a biological product of at least one virus, said method comprising a method according to the invention for the in vitro detection in said biological product of said at least one rare nucleic acid from said virus, and the characterization of said biological product for the presence and/or the level of said at least one virus, wherein said virus is chosen in the group of: adenovirus 5, vesivirus 2117, Porcine circovirus type 1 and 2, Hepatitis C virus, West Nile virus, Hepatitis B virus, Hepatitis E virus, HSV, HCMV, EBV, HHV-6, HHV-7, HHV-8, RSV-A, RSV-B, B19, MVM, PPV, Hepatitis A virus, human polyomavirus BK and JC, MyCV, Human Immunodeficiency virus, Murine leukemia virus, bovine viral diarrhoea virus (BVDV); bovine adenovirus type 3 (BAV3); bovine parvovirus (BPV); bluetongue virus (BTV), BT-2 strain; bovine syncytial virus (BRSV), TN strain; reovirus type 3 (Reo3), Abney strain MLV PPV, influenza B, FluA H3N8 and rabies virus.

In another particular embodiment, the present invention relates to a method for the identification in a biological sample of at least one micro-organism or virus, said method comprising a method for the in vitro detection of at least one rare nucleic acid from a micro-organism or a virus in a sample according to the invention, and a step of identification of at least one micro-organism or virus from the nucleic acid sequence of a molecule of the DNA library generated during said detection method.

In particular, the present disclosure also relates to the use of DSN in a method for the detection of at least one rare nucleic acid molecule in a sample.

In a more particular embodiment, the present invention relates to the use of DSN in a method as defined in the claims for the detection of at least one rare nucleic acid molecule of a micro-organism or a virus in a sample, according to the invention, wherein nucleic acid molecules are contacted with DSN at least before the step of amplifying said nucleic acid molecules.

In particular, the present disclosure also relates to the use of DSN in a method for the detection of at least one rare nucleic acid of a micro-organism or a virus in a sample, according to the invention, wherein nucleic acid molecules are contacted with DSN before and after the step of amplifying said nucleic acid molecules.

In particular, the present disclosure also relates to a kit for the detection in a biological sample of at least one rare nucleic acid molecule from a micro-organism or a virus, wherein said kit comprises a DSN and at least one compound chosen in the group consisting of: buffers, positive control and instructions for use.

In a more particular embodiment, the present invention relates to the use of a kit in a method as defined in the claims which comprises:
a) a DSN,
b) at least one compound chosen in the group consisting of: buffers, positive control and instructions for use, and
c) primers usable for the specific detection of at least one micro-organism or virus.

In particular, a kit disclosed herein comprises:
a) a DSN,
b) at least one compound chosen in the group consisting of: buffers, positive control and instructions for use, and
c) primers usable for the specific detection of at least one micro-organism or virus, wherein said primers are specifically designed for the detection of at least one mycoplasma chosen in the group consisting of: *Mycoplasma hyorhinis*, *Mycoplasma pneumoniae*, *Acheloplasma laidlawii*, *Mycoplasma fermentans*, *Mycoplasma orale*, *Spiroplasma citri*, *Mycoplasma synoviae*, *Mycoplasma gallisepticum* and *Mycoplasma arginini.*

In particular, another kit disclosed herein comprises:
a) a DSN,
b) at least one compound chosen in the group consisting of: buffers, positive control and instructions for use, and
c) primers usable for the specific detection of at least one virus, wherein said primers are specifically designed for the detection of at least one virus chosen in the group consisting of: Adenoviridae, Anelloviridae, Bornaviridae, Bunyaviridae, Caliciviridae, Circoviridae, Coronaviridae , Filoviridae, Flaviviridae, Hepadnaviridae, Hepeviridae, Herpesviridae, Orthomyxoviridae, Papillomaviridae, Paramyxoviridae, Parvoviridae, Picornaviridae, Polyomaviridae, Poxviridae, Reoviridae, Retroviridae, Rhabdoviridae, and Togaviridae.

In particular another kit disclosed herein comprises:
a) a DSN,
b) at least one compound chosen in the group consisting of: buffers, positive control and instructions for use, and
c) primers usable for the specific detection of at least one virus, wherein said primers are specifically designed for the detection of at least one virus chosen in the group consisting of: adenovirus 5, vesivirus 2117, Porcine circovirus type 1 and 2, Hepatitis C virus, West Nile virus, Hepatitis B virus, Hepatitis E virus, HSV, HCMV, EBV, HHV-6, HHV-7, HHV-8, RSV-A, RSV-B, B19, MVM, PPV, Hepatitis A virus, human polyomavirus BK and JC, MyCV, Human Immunodeficiency virus, Murine leukemia virus, bovine viral diarrhoea virus (BVDV); bovine adenovirus type 3 (BAV3); bovine parvovirus (BPV); bluetongue virus (BTV), BT-2 strain; bovine syncytial virus (BRSV), TN strain; reovirus type 3 (Reo3), Abney strain MLV PPV, influenza B, FluA H3N8 and rabies virus.

Particularly, a kit disclosed herein comprises:
- a DSN,
- at least one compound chosen in the group consisting of: buffers, positive control and instructions for use, and
- at least one primer usable for the specific detection of at least one virus, wherein said at least one primer is chosen in the group consisting of:
   a) For detecting a nucleic acid from Ad5: a primer having a nucleotide sequence identical or having at least 90% identity with a sequence chosen in the group consisting of SEQ ID N°1,SEQ ID N°2, and SEQ ID N°3.
   b) For detecting a nucleic acid from BK: a primer having a nucleotide sequence identical or having at least 90% identity with a sequence chosen in the group consisting of SEQ ID N°4,SEQ ID N°5, and SEQ ID N°6.
   c) For detecting a nucleic acid from BVDV: a primer having a nucleotide sequence identical or having at least 90% identity with a sequence chosen in the group consisting of SEQ ID N°7,SEQ ID N°8, and SEQ ID N°9.
   d) For detecting a nucleic acid from HBV: a primer having a nucleotide sequence identical or having at least 90% identity with a sequence chosen in the group consisting of SEQ ID N°10,SEQ ID N° 11, and SEQ ID N° 12.
   e) For detecting a nucleic acid from FluA: a primer having a nucleotide sequence identical or having at least 90% identity with a sequence chosen in the group consisting of SEQ ID N°13,SEQ ID N°14, and SEQ ID N°15.
   f) For detecting a nucleic acid from MLV: a primer having a nucleotide sequence identical or having at least 90% identity with a sequence chosen in the group consisting of SEQ ID N°16,SEQ ID N°17, and SEQ ID N°18.
   g) For detecting a nucleic acid from PPV: a primer having a nucleotide sequence identical or having at least 90% identity with a sequence chosen in the group consisting of SEQ ID N°19,SEQ ID N°20, and SEQ ID N°21.
   h) For detecting a nucleic acid from Reo3: a primer having a nucleotide sequence identical or having at least 90% identity with a sequence chosen in the group consisting of SEQ ID N°22,SEQ ID N°23, and SEQ ID N°24.

The following examples and figures are provided herein for purposes of illustration only and are not intended to be limiting unless otherwise specified.

### LEGENDS OF THE FIGURES

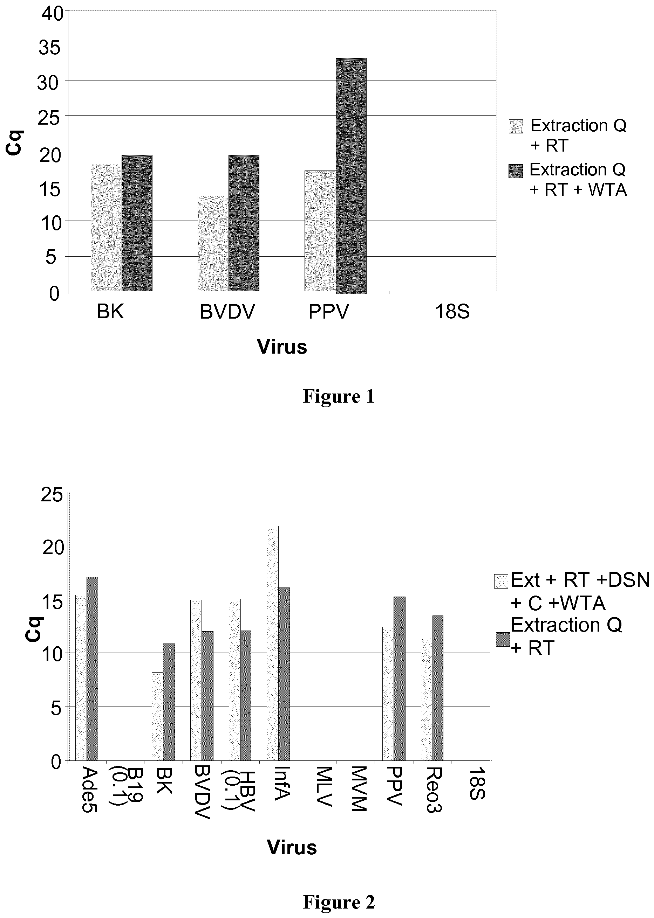

**Figure 1****:** Histogram representation of the difference between gene specific Cq and RNA 18S Cq, with, from left to right, BK, BVDV and PPV. For each virus, the left and clearest bar represent results obtained without amplification, whereas the right and darkest bar represent results obtained with amplification.
**Figure 2****:** Histogram representation of the difference between gene specific Cq and RNA 18S Cq, with, from left to right, Ade5, B19, BK, BVDV, HBV, InfA, MLV, MVM, PPV and Reo3. For each virus, the left and clearest bar represent results obtained with amplification and DSN treatment, whereas the right and darkest bar represent results obtained without amplification.

### EXAMPLES

### Example 1: Evaluation of the number of reads needed for detection

Table 1 summarizes the number of reads that needs to be generated for the detection in a sample prepared from a contaminated host, of one viral particle of PCV1, which is the smallest viral genome known, and of PRV, which represent a bigger viral genome size. Table 1 is based on the hypothesis that said viral genome is present in a sample comprising either 1 µg or 1 ng of total nucleic acid molecules, and that sequences are uniformly amplified when using NGS protocols.

**Table 1**

| | PCV1 | | PRV | |
|---|---|---|---|---|
| Viral genome quantity | 1 | 1 | 1 | 1 |
| Viral genome size (nt) | 1,700 | 1,700 | 170,000 | 170,000 |
| Host DNA quantity (g) | 10⁻⁶ | 10⁻⁹ | 10⁻⁶ | 10⁻⁹ |
| Number of NGS reads needed | 10¹² | 10⁰⁹ | 10¹⁰ | 10⁰⁷ |

The number of reads is dependent of the size of the viral genome, with small genome size increase the number of reads by the order of 2 log10 compared to higher genome size. Although the capacity of NGS machine is increasing, the total number of reads could be too important for the machines capacity.

### Example 2: Effect of DSN treatment on the amplification of rare nucleic acids

Supernatant from human MRC5 cells were obtained and artificially spiked with viruses having different types of genomes, at a virus concentration close to their limit of qPCR detection, the LOD of the corresponding quantitative (RT)-PCR. The following viruses were spiked: human adenovirus type 5 (HADV-5 or Adeno 5, or Ade5; produced and titrated in A 549 cells), parvovirus B19 (B19, World Health Organization stock, NIBSC 99/800, titre expressed in International Units), Human polyomavirus BK (BK ATCC-837), NADL strain of bovine viral diarrhea virus (BVDV-1-NADL or BVDV, produced and titrated in MDBK cells), hepatitis B virus (HBV, WHO stock, NIBSC 97/750, titre expressed in IU), equine influenza virus A (InfA or FluA H3N8, produced in MDCK cells and titrated in genome copies (gc) per ml by quantitative PCR), Moloney Murine leukemia virus (MoMLV ATCC VR-190, produced in NIH/3T3 and titrated in Fg10S+L- cells), porcine parvovirus (PPV, VR-742, produced and titrated on ST cells) and reovirus type 3 (or Reo3, ATCC VR-232, produced and titrated in Vero cells)).

Table 2 summarizes the virus tested, their origin and their dilution as a function of their limit of detection (LOD).

**Table 2**

| Virus | Reference | Concentration (x LOD) |
|---|---|---|
| Adeno 5 | ATCC VR-5 | 1 |
| B19 | NIBSC 99/800 | 1 |
| BK | ATCC VR-837 | 1 |
| BVDV | ATCC VR-534 | 1 |
| HBV | NIBSC 97/750 | 0.1 |
| FluA H3N8 | ATCC VR-317 | 1 |
| MLV | ATCC VR-190 | 0.07 |
| PPV | ATCC VR-742 | 1 |
| reovirus 3 | ATCC VR-232 | 1 |

Specific real time PCR assays were first performed to compare the amplification of rare viral nucleic acid with the amplification of 18S RNA sequences (18S) in the absence of any DSN treatment.

The nucleic acid sequence of the primers used for PCR assays are the following:
- **Ad5 (Van de Pol *et al.,* 2007)**
   Ad5 F: 5'-AACCAGTTGCCGTGAGAG-3' (SEQ ID N°1)
   Ad5 R: 5'-TCGTTAAGCAAGTCCTCGATACAT-3' (SEQ ID N°2)
   Ad5 probe: 5'-(6-Fam)TGGGCGTCGCCAGGCTGTG(Tamra)-3' (SEQ ID N°3)
- **BK (Whiley *et al.,* 2001)**
   BK-F: 5' CACTTTTGGGGGACCTAGT 3' (SEQ ID N°4)
   BK-R: 5' CTCTACAGTAGCAAGGGATGC 3' (SEQ ID N°5)
   BK-Probe: 5' (6-Fam)TCTGAGGCTGCTGCTGCCACAGGATTTT(Tamra) 3' (SEQ ID N°6)
- **BVDV (Bhudevi B., & Weinstock D., 2001)**
   BVDV-F: 5'-TAGCCATGCCCTTAGTAGGAC-3' (SEQ ID N°7)
   BVDV-R : 5'-GACGACTACCCTGTACTCAGG-3' (SEQ ID N°8)
   BVDV-Probe : 5'-(6-Fam)AACAGTGGTGAGTTCGTTGGATGGCTT(Tamra)-3' (SEQ ID N°9)
- **HBV (Zanella *et al.,* 2002)**
   HBV FOR: 5'-CCTATGGGAGTGGGCCTCA-3' (SEQ ID N°10)
   HBV REV: 5'-CCCCAATACCACATCATCCATATA-3' (SEQ ID N°11)
   HBV : 5'-(6-Fam)CACTGAACAAATGGCACTAGTAAACTGAGCCA(Tamra)-3' (SEQ ID N°12)
- **FluA (Watzinger et al., 2004):**
   FluA-F : 5' CATGGAATGGCTAAAGACAAGACC 3' (SEQ ID N°13)
   FluA-R : 5' CCATTTAGGGCATTTTGGACA 3'(SEQ ID N°14)
   FluA-Probe : 5' Fam TTTGTGTTCACGCTCACCGTGCCCA Tamra 3' (SEQ ID N°15)
- **MLV**
   MLV-F : 5'-AAATGAAAGACCCCCGCTG-3' (SEQ ID N°16)
   MLV-R : 5'-TGGTCTCGCTGTTCCTTGG-3' (SEQ ID N°17)
   MLV-Probe : 5'-(6-Fam)CGGGTAGTCAATCACTCAGAGGAGACCCT(Tamra)-3'(SEQ ID N°18)
- **PPV**
   PPVVP2F : 5'-CCAACATGGACACTTAACCACATC-3' (SEQ ID N°19)
   PPVVP2R : 5'-GTTGATTAAATTGTTGCTTTGGAGC-3' (SEQ ID N°20)
   PPV-probe : 5'-(6-Fam) TCACAAGAGCTAGAAAGATACACATTCAATCCACAAAG(Tamra)-3'(SEQ ID N°21)
- **REO3**
   RE03-3F: 5'-GAGATGTTAGGTTTCTGCGTCTAGTG-3' (SEQ ID N°22)
   RE03-R : 5'-AGAATGGGTAATCATCACAATCATACA-3' (SEQ ID N°23)
   RE03-Probe : 5'-(6-Fam)TGCCCCACCGCAGGTCTTAGCTC(Tamra)-3' (SEQ ID N°24)

For B19, the sensitivity of qPCR is described in Cheval *et al.,* (2011).

The nucleic acids extraction procedure was performed with the QIAamp viral RNA mini kit (Qiagen) according to the manufacturer's instruction: 140 µL of samples were extracted, and nucleic acids (DNA and RNA) were eluted in a final volume of 100 µl. Reverse transcription was performed on the eluted fraction using the Reverse Transcriptase SuperScript III (Invitrogen Inc.) according to the manufacturer's instructions.

Samples were treated according to either one of the following methods:
1) Nucleic acids were treated with reverse transcriptase according to manufacturer's instructions (Berthet et al., 2008), without subsequent amplification by RCA. Quantification cycles (Cq) in qPCR assays for BVDV, HBV and PPV were determined, then substracted with Cq of 18S RNA sequences. For each of the three virus tested, the difference between the gene specific matrix Cq and the RNA 18S Cq is comprised between 14 Cq and 18 Cq (Figure 1, left clear bars).
2) Nucleic acids were extracted, treated with reverse transcriptase, and then amplified by RCA. Quantification cycles (Cq) in qPCR assays for BVDV, HBV and PPV were determined, then substracted with Cq of 18S RNA sequences. BVDV, HBV and PPV sequences were less amplified than the 18S sequences. As the PPV sequence was not detected after the RCA amplification, Cq for PPV were defined at 45, corresponding to the end of the PCR program; therefore the difference between Cq is 33. The difference between the matrix Cq and the RNA 18S Cq is comprised between approximately 19 and approximately 33 Cq (Figure 1, right dark bars). Therefore, the difference between rare gene specific and 18S amplification is higher after RCA amplification, indicative of a biased amplification.

In a second step, specific real time PCR assays were performed to compare the amplification of rare viral nucleic acid with the amplification of 18S RNA sequences (18S) after performing a method according to the invention which includes a DSN treatment.

The nucleic acids extraction procedure was performed with the Silica Adembeads kit (Ademtech) according to the manufacturer's instruction: 250 µL of samples were extracted, and nucleic acids (DNA and RNA) were eluted in a final volume of 60 µl. Reverse transcription was performed on 28 µl of the eluted fraction using the Reverse Transcriptase SuperScript III (Invitrogen Inc.) according to the manufacturer's instruction.

Samples were treated according to one of the following methods:
1) Nucleic acids were extracted then treated with reverse transcriptase, without being amplified by RCA. Quantification cycles (Cq) in qPCR assays for Ade5, BK, BVDV, HBV, InfA, PPV and Reo3 were determined, then substracted with Cq of 18S RNA sequences. For each of the virus tested, the difference between the gene specific matrix Cq and the RNA 18S Cq is comprised between 11 Cq and 17 Cq (Figure 2, right dark bars).
2) Nucleic acids were extracted, treated with reverse transcriptase, treated with DSN, then amplified by RCA (method of the invention). Quantification cycles (Cq) for Ade5, B19, BK, BVDV, HBV, InfA, MLV, MVM, PPV and Reo3 were substracted with Cq of 18S RNA (18S) sequences detected in qPCR assays (Figure 2, left clear bars).

When samples were subjected to the DSN and concentration treatment, according to the present invention, the ratio between the viral sequence and the 18S sequence were approximately the same when samples were amplified and not amplified, suggesting an unbiased amplification method. When nucleic acids were submitted to extraction then RT treatment, without any RCA amplification, a difference of approximately 12 and 17 Cq was found between the three matrixes when compared to the 18S sequence. When nucleic acids were submitted to extraction, then RT treatment, then DSN treatment, concentration and RCA amplification, according to a method of the invention, viral sequences show a difference of the three matrices compared to the 18S sequence of approximately 8 and 21 Cq.

The bias amplification phenomenon is based on the low frequency of target, which are far less detected and amplified when compared to higher number of sequence in the same reaction. This leads to the sequencing of the mainly nucleic acids of the host and this could lead to the absence of detection of the contaminant. This could be detrimental in the use of HTS in the detection of low level of contaminant when they are dispersed in biological samples containing high level of host nucleic acids.

The comparison of amplification efficiencies of rare nucleic acid prepared by a method according to the invention shows that this method allows an unbiased amplification of the nucleic acids.

### Example 3: Comparison of the sensitivity of the detection by qPCR, by NGS in a method of prior art and by a method of the present invention

A MRC5 supernatant was artificially spiked with 9 different viruses at a concentration close to the limit of qPCR detection of said virus, as described in example 1. For each virus, two series of samples were prepared:
- virus concentration at 0.8 fold LOD (Mix 1)
- virus concentration at 3 fold LOD (Mix 2)

Virus dilutions are identical to those previously published in Cheval *et al.,* (2011) describing a NGS method (NGS method 1), to allow a comparison of the sensitivity of a method of the present invention (NGS method 2) with the sensitivity of qPCR and of NGS-method 1 described in prior art. The following Table 3 summarizes the name and references of the viruses.

**Table 3**

| | | LOD | |
|---|---|---|---|
| Virus | Reference | Mix 1 | Mix 2 |
| Adenovirus 5 | ATCC VR-5 | 0.8 | 3 |
| BVDV | ATCC VR-534 | 0.8 | 3 |
| FluA H3N8 | ATCC VR-317 | 0.8 | 3 |
| PPV | ATCC VR-742 | 0.8 | 3 |
| reovirus 3 | ATCC VR-232 | 0.8 | 3 |
| SCMV | ATCC VR-677 | 0.8 | 3 |
| Influenza B | ATCC VR-1535 | 0.8 | 3 |
| HBV | NIBSC 97/750 | 0.8 | 3 |
| B19 | NIBSC 99/800 | 0.8 | 3 |
| HCV | NIBSC 96/798 | 0.8 | 3 |

### • NGS Method 1 (Cheval et al., 2011)

A volume of 150 µL of each sample was extracted using a nucleospin RNA virus kit (Macherey-Nagel), and then amplified by the bacteriophage Phi29 polymerase based multiple-displacement-amplification (MDA).

### • NGS Method 2 (present invention)

The nucleic acids extraction procedure was performed with the Silica Adembeads kit (Ademtech) according to the manufacturer's instruction: 250 µL of samples were extracted, and nucleic acids (DNA and RNA) were eluted in a final volume of 60 µl. Reverse transcription was performed on 28 µl of the eluted fraction using the Reverse Transcriptase SuperScript III (Invitrogen Inc.) (Berthet et al, 2008).

The DSN treatment was performed following the RT step: 70 µl of the RT reaction were mixed with 22.75 µl of 4x hybridation buffer (Hepes NaCl 1M EDTA) and heat denatured at 98°C for 3 minutes. The reaction was then maintained for 4 hours at 70°C before the addition of 10.5 µl of prewarmed 2X DSN buffer and 1.75 µl of the DSN enzyme (Evrogen), the reaction was incubated for 10 minutes at 70°C. The reaction was stopped by adding 100 µl of a 2X stop reaction and subjected to a smart extraction for removing the DSN and concentrating the nucleic acids that has been diluted during the preceding steps. According to the manufacturer's instructions, the 200 µl samples were mixed with 200 µl of lysis buffer, 200 µl of binding buffer and 2 µl of Smart D-N-Adembeads (Ademtech). The mixture was mixed twice during 5 minutes incubation and eluted with 10 µl of water for 5 min. The resulting nucleic acid molecules were submitted to ligation and amplified by using the QuantiTect Whole Transcriptome Kit (Qiagen) following the manufacturer's instruction.

### Results

Table 4 presents for each virus the number of detected sequences for NGS method-1 and NGS method-2 (method of present invention) and the detection frequency by qPCR.
Table 4 also summarizes the frequency of detected virus for each method and in each condition (Mix 1 and mix 2).

**Table 4**

| Virus | Mix 1 (0.8 x LOD) | | | Mix 2 (3 x LOD) | | |
|---|---|---|---|---|---|---|
| | qPCR | NGS method 1 | NGS method 2 Method of present invention | qPCR | NGS method 1 | NGS method 2 Method of present invention |
| Adeno 5 | 0/2 | 4 | 60 | *2*/*2* | 13 | 224 |
| BVDV | 2/2 | 1 | 18 | 2/2 | 2 | 56 |
| H3N8 | 0/2 | 0 | 39 | *2*/*2* | *10* | 84 |
| PPV | *2*/*2* | 0 | 5 | 2/2 | *0* | 16 |
| reovirus 3 | *2*/*2* | 0 | 174 | 2/2 | *0* | 154 |
| SCMV | 2/2 | 662 | 5462 | 2/2 | 2142 | 13598 |
| Influenza B | 0/2 | 0 | 68 | *0*/*2* | *4* | 166 |
| HBV | 2/2 | 0 | 21 | 2/2 | *2* | 40 |
| B19 | 1/2 | 6 | 3 | 1/2 | 2 | 8 |
| HCV | *1*/*2* | *0* | 0 | 1/2 | *0* | 0 |
| Detected viruses | 7/10 | 4/10 | 9/10 | 9/10 | 7/10 | 9/10 |

For Mix 1 (0.8 LOD) qPCR, NGS method 1 and NGS method 2 detected respectively 7, 4 and 9 out of 10 viruses. For Mix 2 (3 LOD) qPCR, NGS method 1 and NGS method 2 detected respectively 9, 7 and 9 out of 10 viruses.

By using a method according to the present invention for preparing a nucleic acid sample, 9 of the 10 viruses are detected for both Mix 1 and Mix 2, this shows the efficiency of a method according to the invention. This is not the case with qPCR and when using NGS Method 1. The sensitivity of a method of the present invention is superior to the sensitivity of qPCR. The superior sensitivity of NGS Method 2 to NGS Method 1 demonstrates the technical effect provided by the use of DSN and the concentration step according to a method of the invention.

### Example 4: Detection of viral and bacterial sequences in a biological sample.

Cell supernatant was artificially spiked with four different pathogens, including three different viruses and a mycoplasma specie, at a concentration close to the limit of qPCR detection of said pathogens, as described in the following Table 5. *Mycoplasma Orale* is characterized by a genome composed of double stranded DNA of approximately 680 kpb)

**Table 5**

| Pathogens | Reference | LOD |
|---|---|---|
| MLV | ATCC VR-190 | 1x |
| MVM | Deutsches Krebforschungzentrum, Heidelberg | 1x |
| PCV1 | ATCC CCL-33 | 1x |
| *Mycoplasma Orale* | CIP 104969T | 10cfu/ml |

The protocol used is the same as in Example 3.

The nucleic acids extraction procedure was performed with the Silica Adembeads kit (Ademtech) according to the manufacturer's instruction: 250 µL of samples were extracted, and nucleic acids (DNA and RNA) were eluted in a final volume of 60 µl. Reverse transcription was performed on 28 µl of the eluted fraction using the Reverse Transcriptase SuperScript III (Invitrogen Inc.) (Berthet et al, 2008).

The DSN treatment was performed following the RT step: 70 µl of the RT reaction were mixed with 22.75 µl of 4x hybridation buffer (Hepes NaCl 1M EDTA) and heat denatured at 98°C for 3 minutes. The reaction was then maintained for 4 hours at 70°C before the addition of 10.5 µl of prewarmed 2X DSN buffer and 1.75 µl of the DSN enzyme (Evrogen), the reaction was incubated for 10 minutes at 70°C. The reaction was stopped by adding 100 µl of a 2X stop reaction and subjected to a smart extraction for removing the DSN and concentrating the nucleic acids that has been diluted during the preceding steps. According to the manufacturer's instructions, the 200 µl samples were mixed with 200 µl of lysis buffer, 200 µl of binding buffer and 2 µl of Smart D-N-Adembeads (Ademtech). The mixture was mixed twice during 5 minutes incubation and eluted with 10 µl of water at 70°C for 5 min. The resulting nucleic acid molecules were submitted to ligation and amplified by using the QuantiTect Whole Transcriptome Kit (Qiagen) following the manufacturer's instruction.

The nucleic acid sequence of the primers used for PCR assays are the following:
- PCV1
   PCV1 probe, Sonde TaqMan: 5' Fam AAGCGGCCCGCAACCCCA Tamra 3' (SEQ ID N°25)
   PCV1F, primer sens: 5' CCTCGGCAGCGTCAGTGA 3' (SEQ ID N°26)
   PCV1R, primer reverse: 5' CGGAAGGATTATTAAGGGTGAACAC 3' (SEQ ID N°27)
- MVM
   MvM-Probe, Sonde TaqMan, 5'-(6-Fam) CAAGCCATAGCACAAGCAGTTGGCAA (Tamra) -3' (SEQ ID N°28)
   MvM-F, primer sens : 5'-CCAGCCAGCACAGGCAA-3' (SEQ ID N°29)
   MvM-R, primer antisens : 5'-ACATTGGCTGCATTATAGCAACC-3' (SEQ ID N°30)

### Results

Table 6 presents, for each pathogen, the number of detected sequences by using the method of present invention ("NGS") and the number of cycle threshold (Cp) detected by qPCR.

**Table 6**

| Pathogens | Cp qPCR | NGS |
|---|---|---|
| MLV | 33,19 | 107 |
| MVM | 0 | 7 |
| PCV1 | 40 | 3 |
| *Mycoplasma Orale* | Not done | 3210 |
| Detected pathogen | 2/3 | 4/4 |

The method of the present invention detected the smallest mammalian virus known, PCV-1 and a mycoplasma specie belonging to the bacteria family.

By using a method according to the present invention for preparing a nucleic acid sample, rare microbiological sequences belonging to viral and bacterial sequences can be detected and identified.

### Example 5: Sensitivity of a method of the invention depending on extraction

The sensitivity of a method of the present invention and of qPCR following nucleic acid extraction performed by using different extraction kits, was compared. Two series of samples were prepared in a MRC5 supernatant spiked with a very low level of virus, corresponding to approximately 1 and 0.1 LOD of viral genome and designated respectively as Mix B and Mix C. Table 7 presents the name, origin and dilution of the viruses.

**Table 7**

| Virus | Reference | Mix B concentration (x LOD) | Mix C concentration (x LOD) |
|---|---|---|---|
| Adeno 5 | ATCC VR-5 | 1 | 0.1 |
| B19 | NIBSC 99/800 | 1 | 1 |
| BK | ATCC VR-837 | 1 | 0.1 |
| BVDV | ATCC VR-534 | 1 | 0.1 |
| HBV | NIBSC 97/750 | 0.1 | 0.1 |
| FluA H3N8 | ATCC VR-317 | 1 | 0.1 |
| MLV | ATCC VR-190 | 0.07 | 0.007 |
| PPV | ATCC VR-742 | 1 | 0.1 |
| reovirus 3 | ATCC VR-232 | 1 | 0.1 |

The extraction procedure was the same as presented in Example 3. For the 1 LOD mix and qPCR determination, three extraction kits were evaluated: Qiagen (QIAamp viral RNA mini kit), Macherey Nagel (MN) (NucleoSpin RNA virus) and Ademtech (Silica adembeads extraction kit), whereas for the 0.1 LOD mix two extraction kits were evaluated: Qiagen (QIAamp viral RNA mini kit) and Macherey Nagel (MN) (NucleoSpin RNA virus).

### Results

The following Table 8 presents the number of detected sequences Cq obtained by performing qPCR after using each of the extraction kits, and the number of different sequences detected by using a method according to the invention. For qPCR detection, bold and underlined characters are indicative of the detection of duplicates of the virus sequence, whereas normal characters indicate that only one sequence was detected.

**Table 8**

| Virus | Mix B (1 LOD) | | | | Mix C (0.1 LOD) | | |
|---|---|---|---|---|---|---|---|
| | qPCR Cq | | | Method of present invention | qPCR Cq | | Method of present invention |
| | Qiagen | MN | Ademtech | | Qiagen | MN | |
| Adeno 5 | 39 | **39** | **39** | 73 | *0* | 40 | 5 |
| B19 | 38 | **37** | 37 | 3 | 37 | 38 | 14 |
| BK | **35** | **33** | **33** | 2143 | 38 | **37** | 539 |
| BVDV | **35** | **35** | **34** | 120 | 37 | **38** | 24 |
| HBV | 37 | 37 | **34** | 6 | *0* | 37 | 7 |
| FluA | 39 | **37** | **38** | 66 | 40 | *0* | 6 |
| MLV | 43 | 40 | *0* | 0 | *0* | *0* | *0* |
| PPV | **38** | **37** | **37** | 129 | *0* | 38 | 21 |
| REO3 | **38** | **40** | **38** | 218 | *0* | 40 | 88 |
| Detected virus | 4 to 9 /9 | 7 to 9 /9 | 7 to 8 /9 | 9/9 | 0 to 4 /9 | 2 to 7 /9 | 8/9 |

### Conclusion

All three kits show similar detection level, although a slighter sensitivity can be attributed to Macherey Nagel and Ademtech kits. The sample preparation with a method according to the invention allows the detection of 8 out of 9 viruses detected in the Mix B (1 LOD), showing that the method of the invention is at least as sensitive as qPCR for this amount of contaminating virus.

In mix C (0.1 LOD), 8 out of 9 viruses are detected by using a method of the invention. The only undetected virus corresponds to a MLV spike at a very low level corresponding to 0.007 LOD. With qPCR determination, the best extraction method (MN) allows the detection of 7 out of 9 viruses, with 5 viruses detected with a frequency of 50%, confirming the higher sensitivity of the method according to the invention when compared to qPCR.

### BIBLIOGRAPHIC REFERENCES

Gardner et al., (2003) J Clin Microbiol. 2003 Jun;41(6):2417-27.
Barzon et al., (2011) Int J Mol Sci. 2011; 12(11): 7861-7884.
Vries et al., (2010), PLoS One. 2011 Jan 24;6(1):e16118.
Cheval et al., J. Clin. Microbiol., Sept 2011, 3268-75 (2011)
Rodrigue et al., PLoS ONE, Sept. 2009, vol.4, Issue 9, (2009)
Maniatis T. et al. Molecular cloning, a Laboratory Manual, Cold Spring Harbor Laboratory, Cold Spring Harbor, N.Y., Edition 1999.
Shagin et al., Genome Res. Dec;12(12):1935-42 (2002)
Zhulidov et al., Nucleic Acids Res. Feb 18;32(3):e37 (2004)
Bogdanova et al., Methods MolBiol.;729:85-98. (2011a)
Christodoulou et al., Curr Protoc Mol Biol. Apr; Chapter 4:Unit4.12 (2011)
Berthet et al., (2008) BMC Mol Biol. 2008; 9: 77.
Van de Pol A. C., et al., (2007) J. Clin. Microbiol 45:2260-2262.
Whiley DM et al., (2001) J Clin Microbiol. Dec;39(12):4357-61.
Bhudevi B., and Weinstock D., (2001) Vet. Microbiol 83:1-10.
Zanella I, et al., (2002) J Med Virol. 2002 Dec;68(4):494-9.
Watzinger F. et al., (2004) J. Clin. Microbiol 42:5189-5198.

## Claims

1. Method for the in vitro detection in a biological sample of the presence of at least one rare nucleic acid from a micro-organism or a virus, said method comprising the steps of:
a) extracting the nucleic molecules from a biological sample, said nucleic acid molecules comprising double strand nucleic acid molecules, single strand nucleic acid molecules or a combination thereof,
b) inducing the denaturation and the renaturation of the double strand nucleic acid molecules of step a),
c) contacting the product of step b) with a duplex specific nuclease (DSN),
d) inactivating and/or extracting said DSN from the product of step c) and concentrating the nucleic acid molecules,
e) amplificating the nucleic acid molecules of the product of step d),
f) preparing a DNA library from the product of step e),
g) determining the nucleic acid sequence of molecules of the DNA library of step f) and detecting, from said nucleic acid sequence, at least one rare nucleic acid from a micro-organism or a virus,
wherein the biological sample is prepared from a material chosen in the group consisting of: a human biological fluid or tissue, an animal biological fluid or tissue, a culture of a human or an animal biological fluid or tissue, a culture of human, animal, fungi and unicellular eukaryotes cells, prokaryotic cells or a combination thereof, or a bio-therapeutic product, preferably chosen in the group consisting of: recombinant protein, monoclonal antibody, vaccine, cell therapy product, gene therapy product, products of human or animal origin such as blood-derived products, heparin, hyaluronic acid and collagen, or cells from a cell culture infected by a virus, cells from a hybridoma production such as mouse/human hybridomas used for the production of monoclonal antibodies, and cells susceptible to be used for the production of a recombinant protein, or a cell bank, preferably chosen in the group consisting of: a master cell bank, a working cell bank and a production cell bank.

2. Method according to claim 1, wherein in step a) said nucleic acid molecules comprise DNA molecules, RNA molecules or a combination thereof, and wherein extracting the nucleic acid molecules is followed by the reverse transcription of the RNA molecules of step a).

3. Method according to claim 1 or 2, wherein in step e) amplificating the nucleic acids molecules is performed by rolling circle amplification (RCA).

4. Method according to any one of claim 1 to 3, wherein said micro-organism is chosen in the group consisting of: Gram positive bacteria, Gram negative bacteria, mycoplasmas, yeasts and fungi.

5. Method according to claim 4, wherein said micro-organism is a mycoplasma chosen in the group consisting of: *Mycoplasma hyorhinis, Mycoplasma pneumoniae*, *Acheloplasma laidlawii*, *Mycoplasma fermentans*, *Mycoplasma orale*, *Spiroplasma citri*, *Mycoplasma synoviae*, *Mycoplasma gallisepticum* and *Mycoplasma arginini.*

6. Method according to any one of claims claim 1 to 3, wherein said at least one rare nucleic acid molecule is a nucleic acid molecule of a virus.

7. Method according to claim 6, wherein said virus is chosen in the group consisting of: double-strand DNA virus, single-strand DNA virus, double-strand RNA virus and single-strand RNA virus, segmented virus and non-segmented virus.

8. Method according to claim 7, wherein said virus is chosen in the group consisting of: human virus, simian virus, rodent virus, equine virus, bovine virus, porcine virus, insect virus and bacteriophage.

9. Method according to claim 7 or 8, wherein said virus is chosen in the group consisting of: Hepatitis C virus (HCV), hepatitis B virus (HBV), B19, adenovirus 5, BK, BVDV, FluA H3N8, MLV, PPV, reovirus 3 and influenza B virus.

10. Method for the characterization of a biological sample, said method comprising the detection of at least one rare nucleic acid from a micro-organism or virus according to any one of claims 1 to 9 and the characterization of said biological sample for the presence and/or level of said at least one micro-organism or virus.

11. Method for the evaluation of a manufacturing process for the preparation of a biological sample, said method comprising the detection in said biological sample of at least one rare nucleic acid from a micro-organism or virus according to any one of claims 1 to 9, wherein said detection of said rare nucleic acid from a micro-organism or virus is performed at at least two different stages of said manufacturing process.

12. Use in the method of claim 1 of a DSN for the detection in a biological sample of at least one rare nucleic acid from a micro-organism or a virus, wherein the biological sample is prepared from a material chosen in the group consisting of: a human biological fluid or tissue, an animal biological fluid or tissue, a culture of a human or an animal biological fluid or tissue, a culture of human, animal, fungi and unicellular eukaryotes cells, prokaryotic cells or a combination thereof, or a bio-therapeutic product, preferably chosen in the group consisting of: recombinant protein, monoclonal antibody, vaccine, cell therapy product, gene therapy product, products of human or animal origin such as blood-derived products, heparin, hyaluronic acid and collagen, or cells from a cell culture infected by a virus, cells from a hybridoma production such as mouse/human hybridomas used for the production of monoclonal antibodies, and cells susceptible to be used for the production of a recombinant protein, or a cell bank, preferably chosen in the group consisting of: a master cell bank, a working cell bank and a production cell bank.

13. Use according to claim 12, in a method comprising a step of amplifying nucleic acid molecules, wherein the use of said DSN is performed at least before said step of amplifying nucleic acid molecules.

14. Use of a kit in the method of claim 1 for the detection in a biological sample of at least one rare nucleic acid from a micro-organism or a virus, comprising DSN and at least one compound chosen in the group consisting of: buffers, positive control and instructions for use, wherein the biological sample is prepared from a material chosen in the group consisting of: a human biological fluid or tissue, an animal biological fluid or tissue, a culture of a human or an animal biological fluid or tissue, a culture of human, animal, fungi and unicellular eukaryotes cells, prokaryotic cells or a combination thereof, or a bio-therapeutic product, preferably chosen in the group consisting of: recombinant protein, monoclonal antibody, vaccine, cell therapy product, gene therapy product, products of human or animal origin such as blood-derived products, heparin, hyaluronic acid and collagen, or cells from a cell culture infected by a virus, cells from a hybridoma production such as mouse/human hybridomas used for the production of monoclonal antibodies, and cells susceptible to be used for the production of a recombinant protein, or a cell bank, preferably chosen in the group consisting of: a master cell bank, a working cell bank and a production cell bank.

15. Use according to claim 14 of a kit in the method of claim 1, wherein the kit comprises the following elements:
a) a DSN,
b) at least one compound chosen in the group consisting of: buffers, positive control and instructions for use, and
c) at least one primer able to detect specifically said at least one micro-organism or virus.

## Patentansprüche

1. Verfahren für die in vitro-Feststellung des Vorhandenseins von mindestens einer seltenen Nukleinsäure von einem Mikroorganismus oder einem Virus in einer biologischen Probe, wobei das Verfahren die folgenden Schritte umfasst:
a) Extrahieren der Nukleinsäuremoleküle aus einer biologischen Probe, wobei die Nukleinsäuremoleküle doppelsträngige Nukleinsäuremoleküle, einzelsträngige Nukleinsäuremoleküle oder eine Kombination derselben umfassen,
b) Auslösen der Denaturierung und Renaturierung der doppelsträngigen Nukleinsäuremoleküle von Schritt a),
c) Kontaktieren des Produktes von Schritt b) mit einer duplex-spezifischen Nuklease (DSN),
d) Inaktivieren und/oder Extrahieren der DSN aus dem Produkt von Schritt c) und Konzentrieren der Nukleinsäuremoleküle,
e) Amplifizieren der Nukleinsäuremoleküle des Produktes von Schritt d),
f) Erstellen einer DNA-Bibliothek aus dem Produkt von Schritt e),
g) Bestimmen der Nukleinsäuresequenz von Molekülen der DNA-Bibliothek von Schritt f) und Feststellen von mindestens einer seltenen Nukleinsäuren von einem Mikroorganismus oder einem Virus aus der Nukleinsäuresequenz,
wobei die biologische Probe aus einem Material hergestellt wird, das in der Gruppe gewählt wird, die besteht aus: einem menschlichen biologischen Fluid oder Gewebe, einem tierischen biologischen Fluid oder Gewebe, einer Kultur eines menschlichen oder eines tierischen biologischen Fluids oder Gewebes, einer Kultur von menschlichen, tierischen, pilzlichen oder einzelligen Eukaryoten-Zellen, prokaryotischen Zellen oder einer Kombination derselben, oder einem biotherapeutischen Produkt, vorzugsweise gewählt in der Gruppe, die besteht aus: rekombinantes Protein, monoclonalen Antikörpern, Vakzine, Zelltherapieprodukt, Gentherapieprodukt, Produkte von menschlichem oder tierischem Ursprung, wie zum Beispiel von Blut stammenden Produkten, Heparin, Hyaluronsäure und Kollagen, oder Zellen aus einer Zellkultur, die mit einem Virus infiziert ist, Zellen, von einem Hybridom erzeugt, wie zum Beispiel Maus-/menschliche Hybridome, die für die Herstellung von monoclonalen Antikörpern verwendet werden, und Zellen, die empfindlich sind, um für die Herstellung eines rekombinanten Proteins verwendet zu werden, oder aus einer Zellbank, vorzugsweise gewählt in der Gruppe, die besteht aus: einer Master-Zellbank, einer Arbeitszellbank und einer Produktionszellbank.

2. Verfahren nach Anspruch 1, wobei in Schritt a) die Nukleinsäuremoleküle DNA-Moleküle, RNA-Moleküle oder eine Kombination derselben umfassen, und wobei dem Extrahieren der Nukleinsäuremoleküle die reverse Transkription der RNA-Moleküle von Schritt a) folgt.

3. Verfahren nach Anspruch 1 oder 2, wobei in Schritt e) die Amplifikation der Nukleinsäuremoleküle durch Rolling-Circle-Amplifikation (RCA) ausgeführt wird.

4. Verfahren nach einem der Ansprüche 1 bis 3, wobei der Mikroorganismus in der Gruppe gewählt wird, die besteht aus: grampositiven Bakterien, gramnegativen Bakterien, Mycoplasmen, Hefen und Pilzen.

5. Verfahren nach Anspruch 4, wobei der Mikroorganismus ein Mycoplasma ist, das in der Gruppe gewählt wird, die besteht aus: *Mycoplasma hyorhinis*, *Mycoplasma pneumoniae*, *Acheloplasma laidlawii, Mycoplasma fermentans*, *Mycoplasma orale, Spiroplasma citri, Mycoplasma synoviae, Mycoplasma gallisepticum* and *Mycoplasma arginini.*

6. Verfahren nach einem der Ansprüche 1 bis 3, wobei das mindestens eine seltene Nukleinsäuremolekül ein Nukleinsäuremolekül eines Virus ist.

7. Verfahren nach Anspruch 6, wobei das Virus in der Gruppe gewählt wird, die besteht aus: doppelsträngiges DNA-Virus, einzelsträngiges DNA-Virus, doppelsträngiges RNA-Virus und einzelsträngiges RNA-Virus, segmentiertes Virus und nicht-segmentiertes Virus.

8. Verfahren nach Anspruch 7, wobei das Virus in der Gruppe gewählt wird, die besteht aus: menschlichem Virus, Simianvirus, Nagetiervirus, Pferdevirus, Rindervirus, Schweinevirus, Insektenvirus und Bakteriophagen.

9. Verfahren nach Anspruch 7 oder 8, wobei das Virus in der Gruppe gewählt wird, die besteht aus: Hepatitis C-Virus (HCV), Hepatitis B-Virus (HBV), B19, Adenovirus 5, BK, BVDV, FluA H3N8, MLV, PPV, Reovirus 3 und Influenza B-Virus.

10. Verfahren zur Charakterisierung einer biologischen Probe, wobei das Verfahren die Feststellung von mindestens einer seltenen Nukleinsäure von einem Mikroorganismus oder Virus nach einem der Ansprüche 1 bis 9 und die Charakterisierung der biologischen Probe für das Vorhandensein und/oder die Menge des mindestens einen Mikroorganismus oder Virus umfasst.

11. Verfahren zur Bewertung eines Herstellungsprozesses für die Präparation einer biologischen Probe, wobei das Verfahren die Feststellung, in der biologischen Probe, von mindestens einer seltenen Nukleinsäure von einem Mikroorganismus oder Virus nach einem der Ansprüche 1 bis 9 umfasst, wobei die Feststellung der seltenen Nukleinsäure von einem Mikroorganismus oder Virus in mindestens zwei Stufen des Herstellungsprozesses ausgeführt wird.

12. Verwendung, bei dem Verfahren nach Anspruch 1, einer DSN für die Feststellung, in einer biologischen Probe, von mindestens einer seltenen Nukleinsäure von einem Mikroorganismus oder einem Virus, wobei die biologische Probe aus einem Material präpariert wird, das in der Gruppe gewählt wird, die besteht aus: einem menschlichen biologischen Fluid oder Gewebe, einem tierischen biologischen Fluid oder Gewebe, einer Kultur eines menschlichen oder eines tierischen biologischen Fluids oder Gewebes, einer Kultur von menschlichen, tierischen, pilzlichen oder einzelligen Eukaryoten-Zellen, prokaryotischen Zellen oder einer Kombination derselben, oder einem biotherapeutischen Produkt, vorzugsweise gewählt in der Gruppe, die besteht aus: rekombinantem Protein, monoclonalen Antikörpern, Vakzine, Zelltherapieprodukt, Gentherapieprodukt, Produkte von menschlichem oder tierischem Ursprung, wie zum Beispiel von Blut stammenden Produkten, Heparin, Hyaluronsäure und Kollagen, oder Zellen aus einer Zellkultur, die mit einem Virus infiziert ist, Zellen, von einem Hybridom erzeugt, wie zum Beispiel Maus-/menschliche Hybridome, die für die Herstellung von monoclonalen Antikörpern verwendet werden, und Zellen, die empfindlich sind, um für die Herstellung eines rekombinanten Proteins verwendet zu werden, oder aus einer Zellbank, vorzugsweise gewählt in der Gruppe, die besteht aus: einer Master-Zellbank, einer Arbeitszellbank und einer Produktionszellbank.

13. Verwendung nach Anspruch 12, bei einem Verfahren, das einen Schritt des Amplifizierens von Nukleinsäuremolekülen umfasst, wobei die Verwendung der DSN zumindest vor dem Schritt des Amplifizierens der Nukleinsäuremoleküle ausgeführt wird.

14. Verwendung eines Ausrüstungssatzes bei dem Verfahren nach Anspruch 1 für die Feststellung, in einer biologischen Probe, von mindestens einer seltenen Nukleinsäure von einem Mikroorganismus oder einem Virus, die DSN und mindestens eine Verbindung enthält, die in der Gruppe gewählt wird, bestehend aus: Puffer, Positivkontrolle und Anweisungen zur Verwendung, wobei die biologische Probe aus einem Material präpariert wird, das in der Gruppe gewählt wird, die besteht aus: einem menschlichen biologischen Fluid oder Gewebe, einem tierischen biologischen Fluid oder Gewebe, einer Kultur eines menschlichen oder eines tierischen biologischen Fluids oder Gewebes, einer Kultur von menschlichen, tierischen, pilzlichen oder einzelligen Eukaryoten-Zellen, prokaryotischen Zellen oder einer Kombination derselben, oder einem biotherapeutischen Produkt, vorzugsweise gewählt in der Gruppe, die besteht aus: rekombinantem Protein, monoclonalen Antikörpern, Vakzine, Zelltherapieprodukt, Gentherapieprodukt, Produkte von menschlichem oder tierischem Ursprung, wie zum Beispiel von Blut stammenden Produkten, Heparin, Hyaluronsäure und Kollagen, oder Zellen aus einer Zellkultur, die mit einem Virus infiziert ist, Zellen, von einem Hybridom erzeugt, wie zum Beispiel Maus-/menschliche Hybridome, die für die Herstellung von monoclonalen Antikörpern verwendet werden, und Zellen, die empfindlich sind, um für die Herstellung eines rekombinanten Proteins verwendet zu werden, oder aus einer Zellbank, vorzugsweise gewählt in der Gruppe, die besteht aus: einer Master-Zellbank, einer Arbeitszellbank und einer Produktionszellbank.

15. Verwendung eines Ausrüstungssatzes nach Anspruch 14 bei dem Verfahren nach Anspruch 1, wobei der Ausrüstungsansatz die folgenden Elemente umfasst:
a) eine DSN,
b) mindestens eine Verbindung, die in der Gruppe gewählt wird, bestehend aus: Puffer, Positivkontrolle und Anweisungen zur Verwendung, und
c) mindestens ein Primer, der in der Lage ist, den mindestens einen Mikroorganismus oder Virus spezifisch festzustellen.

## Revendications

1. Procédé pour la détection in vitro dans un échantillon biologique de la présence d'au moins un acide nucléique rare provenant d'un microorganisme ou d'un virus, ledit procédé comprenant les étapes suivantes :
a) extraction des molécules d'acide nucléique à partir d'un échantillon biologique, lesdites molécules d'acide nucléique comprenant des molécules d'acide nucléique double brin, des molécules d'acide nucléique simple brin ou une combinaison de celles-ci,
b) induction de la dénaturation et de la renaturation des molécules d'acide nucléique double brin de l'étape a),
c) mise en contact du produit de l'étape b) avec une nucléase spécifique de duplex (DSN),
d) inactivation et/ou extraction de ladite DSN à partir du produit de l'étape c) et concentration des molécules d'acide nucléique,
e) amplification des molécules d'acide nucléique du produit de l'étape d),
f) préparation d'une bibliothèque d'ADN à partir du produit de l'étape e),
g) détermination de la séquence d'acide nucléique de molécules de la bibliothèque d'ADN de l'étape f) et détection, à partir de ladite séquence d'acide nucléique, d'au moins un acide nucléique rare provenant d'un microorganisme ou d'un virus,
dans lequel l'échantillon biologique est préparé à partir d'un matériel choisi dans le groupe constitué par : un tissu ou fluide biologique humain, un tissu ou fluide biologique animal, une culture d'un tissu ou fluide biologique humain ou animal, une culture de cellules eucaryotes humaines, animales, fongiques et unicellulaires, de cellules procaryotes, ou d'une combinaison de celles-ci, ou un produit bio-thérapeutique, de préférence choisi dans le groupe constitué par : une protéine recombinée, un anticorps monoclonal, un vaccin, un produit de thérapie cellulaire, un produit de thérapie génique, les produits d'origine humaine ou animale tels que les produits dérivés du sang, l'héparine, l'acide hyaluronique et le collagène, ou les cellules provenant d'une culture cellulaire infectée par un virus, les cellules provenant d'une production d'hybridomes telles que des hybridomes de souris/humain utilisés pour la production d'anticorps monoclonaux, et les cellules susceptibles d'être utilisées pour la production d'une protéine recombinée, ou une banque de cellules, de préférence choisie dans le groupe constitué par : une banque de cellules primaire, une banque de cellules de travail et une banque de cellules de production.

2. Procédé selon la revendication 1, dans lequel, dans l'étape a), lesdites molécules d'acide nucléique comprennent des molécules d'ADN, des molécules d'ARN ou une combinaison de celles-ci, et dans lequel l'extraction des molécules d'acide nucléique est suivie d'une transcription inverse des molécules d'ARN de l'étape a).

3. Procédé selon la revendication 1 ou 2, dans lequel, dans l'étape e), l'amplification des molécules d'acide nucléique est effectuée par amplification par cercle roulant (pour 'rolling circle amplification' RCA) .

4. Procédé selon l'une quelconque des revendications 1 à 3, dans lequel ledit micro-organisme est choisi dans le groupe constitué par : les bactéries Gram positives, les bactéries Gram négatives, les mycoplasmes, les levures et les champignons.

5. Procédé selon la revendication 4, dans lequel ledit micro-organisme est un mycoplasme choisi dans le groupe constitué par : *Mycoplasma hyorhinis*, *Mycoplasma pneumoniae*, *Acheloplasma laidlawii*, *Mycoplasma fermentans*, *Mycoplasma orale*, *Spiroplasma citri*, *Mycoplasma synoviae*, *Mycoplasma gallisepticum* et *Mycoplasma arginini.*

6. Procédé selon l'une quelconque des revendications 1 à 3, dans lequel ladite au moins une molécule d'acide nucléique rare est une molécule d'acide nucléique d'un virus.

7. Procédé selon la revendication 6, dans lequel ledit virus est choisi dans le groupe constitué par : un virus à ADN double brin, un virus à ADN simple brin, un virus à ARN double brin et un virus à ARN simple brin, un virus segmenté et un virus non segmenté.

8. Procédé selon la revendication 7, dans lequel ledit virus est choisi dans le groupe constitué par : un virus humain, un virus simien, un virus de rongeur, un virus équin, un virus bovin, un virus porcin, un virus d'insecte et un bactériophage.

9. Procédé selon la revendication 7 ou 8, dans lequel ledit virus est choisi dans le groupe constitué par : le virus de l'hépatite C (VHC), le virus de l'hépatite B (VHB), B19, l'adénovirus 5, BK, BVDV, le virus grippal A H3N8, MLV, PPV, le réovirus 3 et le virus grippal B.

10. Procédé pour la caractérisation d'un échantillon biologique, ledit procédé comprenant la détection d'au moins un acide nucléique rare provenant d'un microorganisme ou virus selon l'une quelconque des revendications 1 à 9, et la caractérisation dudit échantillon biologique par la présence et/ou le niveau dudit au moins un microorganisme ou virus.

11. Procédé pour l'évaluation d'un procédé de fabrication de la préparation d'un échantillon biologique, ledit procédé comprenant la détection dans ledit échantillon biologique d'au moins un acide nucléique rare provenant d'un microorganisme ou virus selon l'une quelconque des revendications 1 à 9, dans lequel ladite détection dudit acide nucléique rare provenant d'un microorganisme ou virus est effectuée à au moins deux stades différents dudit procédé de fabrication.

12. Utilisation, dans le procédé de la revendication 1, d'une DSN pour la détection dans un échantillon biologique d'au moins un acide nucléique rare provenant d'un microorganisme ou d'un virus, dans laquelle l'échantillon biologique est préparé à partir d'un matériau choisi dans le groupe constitué par : un tissu ou fluide biologique humain, un tissu ou fluide biologique animal, une culture d'un tissu ou fluide biologique humain ou animal, une culture de cellules eucaryotes humaines, animales, fongiques et unicellulaires, de cellules procaryotes, ou d'une combinaison de celles-ci, ou un produit bio-thérapeutique, de préférence choisi dans le groupe constitué par : une protéine recombinée, un anticorps monoclonal, un vaccin, un produit de thérapie cellulaire, un produit de thérapie génique, les produits d'origine humaine ou animale tels que les produits dérivés du sang, l'héparine, l'acide hyaluronique et le collagène, ou les cellules provenant d'une culture cellulaire infectée par un virus, les cellules provenant d'une production d'hybridomes telles que des hybridomes de souris/humain utilisés pour la production d'anticorps monoclonaux, et les cellules susceptibles d'être utilisées pour la production d'une protéine recombinée, ou une banque de cellules, de préférence choisie dans le groupe constitué par : une banque de cellules primaire, une banque de cellules de travail et une banque de cellules de production.

13. Utilisation selon la revendication 12, dans un procédé comprenant une étape d'amplification de molécules d'acide nucléique, dans laquelle l'utilisation de ladite DSN est effectuée au moins avant ladite étape d'amplification de molécules d'acide nucléique.

14. Utilisation d'un kit dans le procédé de la revendication 1 pour la détection dans un échantillon biologique d'au moins un acide nucléique rare provenant d'un microorganisme ou d'un virus, comprenant une DSN et au moins un composé choisi dans le groupe constitué par : les tampons, les contrôles positifs et les instructions d'utilisation, dans laquelle l'échantillon biologique est préparé à partir d'un matériau choisi dans le groupe constitué par : un tissu ou fluide biologique humain, un tissu ou fluide biologique animal, une culture d'un tissu ou fluide biologique humain ou animal, une culture de cellules eucaryotes humaines, animales, fongiques et unicellulaires, de cellules procaryotes, ou d'une combinaison de celles-ci, ou un produit bio-thérapeutique, de préférence choisi dans le groupe constitué par : une protéine recombinée, un anticorps monoclonal, un vaccin, un produit de thérapie cellulaire, un produit de thérapie génique, les produits d'origine humaine ou animale tels que les produits dérivés du sang, l'héparine, l'acide hyaluronique et le collagène, ou les cellules provenant d'une culture cellulaire infectée par un virus, les cellules provenant d'une production d'hybridomes telles que des hybridomes de souris/humain utilisés pour la production d'anticorps monoclonaux, et les cellules susceptibles d'être utilisées pour la production d'une protéine recombinée, ou une banque de cellules, de préférence choisie dans le groupe constitué par : une banque de cellules primaire, une banque de cellules de travail et une banque de cellules de production.

15. Utilisation selon la revendication 14 d'un kit dans le procédé de la revendication 1, dans laquelle le kit comprend les éléments suivants :
a) une DSN,
b) au moins un composé choisi dans le groupe constitué par : les tampons, les contrôles positifs et les instructions d'utilisation, et
c) au moins une amorce capable de détecter spécifiquement ledit au moins un microorganisme ou virus.


### SEQUENCE LISTING

<110> Texcell
<120> Detection of rare microbiological nucleic acids
<130> 366285D32077
<150> EP13306360.2
   <151> 2013-10-01
<160> 30
<170> PatentIn version 3.5
<210> 1
   <211> 18
   <212> DNA
   <213> artificial
<220>
   <223> Ad5 primer-F
<400> 1
   aaccagttgc cgtgagag 18
<210> 2
   <211> 24
   <212> DNA
   <213> artificial
<220>
   <223> Ad5 R-primer
<400> 2
   tcgttaagca agtcctcgat acat 24
<210> 3
   <211> 19
   <212> DNA
   <213> artificial
<220>
   <223> Ad5 probe
<400> 3
   tgggcgtcgc caggctgtg 19
<210> 4
   <211> 19
   <212> DNA
   <213> artificial
<220>
   <223> BK F-primer
<400> 4
   cacttttggg ggacctagt 19
<210> 5
   <211> 21
   <212> DNA
   <213> artificial
<220>
   <223> BK R-primer
<400> 5
   ctctacagta gcaagggatg c 21
<210> 6
   <211> 28
   <212> DNA
   <213> artificial
<220>
   <223> BK probe
<400> 6
   tctgaggctg ctgctgccac aggatttt 28
<210> 7
   <211> 21
   <212> DNA
   <213> artificial
<220>
   <223> BVDV F-primer
<400> 7
   tagccatgcc cttagtagga c 21
<210> 8
   <211> 21
   <212> DNA
   <213> artificial
<220>
   <223> BVDV R-primer
<400> 8
   gacgactacc ctgtactcag g 21
<210> 9
   <211> 27
   <212> DNA
   <213> artificial
<220>
   <223> BVDV probe
<400> 9
   aacagtggtg agttcgttgg atggctt 27
<210> 10
   <211> 19
   <212> DNA
   <213> artificial
<220>
   <223> HBV F-primer
<400> 10
   cctatgggag tgggcctca 19
<210> 11
   <211> 24
   <212> DNA
   <213> artificial
<220>
   <223> HBV R-primer
<400> 11
   ccccaatacc acatcatcca tata 24
<210> 12
   <211> 32
   <212> DNA
   <213> artificial
<220>
   <223> HBV probe
<400> 12
   cactgaacaa atggcactag taaactgagc ca 32
<210> 13
   <211> 24
   <212> DNA
   <213> artificial
<220>
   <223> FluA F-primer
<400> 13
   catggaatgg ctaaagacaa gacc 24
<210> 14
   <211> 21
   <212> DNA
   <213> artificial
<220>
   <223> FluA R-primer
<400> 14
   ccatttaggg cattttggac a 21
<210> 15
   <211> 25
   <212> DNA
   <213> artificial
<220>
   <223> Flu probe
<400> 15
   tttgtgttca cgctcaccgt gccca 25
<210> 16
   <211> 19
   <212> DNA
   <213> artificial
<220>
   <223> MLV F-primer
<400> 16
   aaatgaaaga cccccgctg 19
<210> 17
   <211> 19
   <212> DNA
   <213> artificial
<220>
   <223> MLV R-primer
<400> 17
   tggtctcgct gttccttgg 19
<210> 18
   <211> 29
   <212> DNA
   <213> artificial
<220>
   <223> MLV probe
<400> 18
   cgggtagtca atcactcaga ggagaccct 29
<210> 19
   <211> 24
   <212> DNA
   <213> artificial
<220>
   <223> PPVVP2F F-primer
<400> 19
   ccaacatgga cacttaacca catc 24
<210> 20
   <211> 25
   <212> DNA
   <213> artificial
<220>
   <223> PPVVP2R R-primer
<400> 20
   gttgattaaa ttgttgcttt ggagc 25
<210> 21
   <211> 38
   <212> DNA
   <213> artificial
<220>
   <223> PPV probe
<400> 21
   tcacaagagc tagaaagata cacattcaat ccacaaag 38
<210> 22
   <211> 26
   <212> DNA
   <213> artificial
<220>
   <223> Reo3 F-primer
<400> 22
   gagatgttag gtttctgcgt ctagtg 26
<210> 23
   <211> 27
   <212> DNA
   <213> artificial
<220>
   <223> Reo3 R-primer
<400> 23
   agaatgggta atcatcacaa tcataca 27
<210> 24
   <211> 23
   <212> DNA
   <213> artificial
<220>
   <223> Reo3 probe
<400> 24
   tgccccaccg caggtcttag ctc 23
<210> 25
   <211> 18
   <212> DNA
   <213> Artificial Sequence
<220>
   <223> PCV1 probe
<400> 25
   aagcggcccg caacccca 18
<210> 26
   <211> 18
   <212> DNA
   <213> Artificial Sequence
<220>
   <223> PCV1F
<400> 26
   cctcggcagc gtcagtga 18
<210> 27
   <211> 25
   <212> DNA
   <213> Artificial Sequence
<220>
   <223> PCV1R
<400> 27
   cggaaggatt attaagggtg aacac 25
<210> 28
   <211> 26
   <212> DNA
   <213> Artificial Sequence
<220>
   <223> MvM probe
<400> 28
   caagccatag cacaagcagt tggcaa 26
<210> 29
   <211> 26
   <212> DNA
   <213> Artificial Sequence
<220>
   <223> MvM-F
<400> 29
   caagccatag cacaagcagt tggcaa 26
<210> 30
   <211> 23
   <212> DNA
   <213> Artificial Sequence
<220>
   <223> MvM-R
<400> 30
   acattggctg cattatagca acc 23